# EUROPEAN PATENT SPECIFICATION

(11) **EP 0 643 678 B1**
(45) Date of publication and mention of the grant of the patent: **12.02.1997**
(21) Application number: 93915202.1
(22) Date of filing: 03.06.1993
(51) Int. Cl.: C07C 15/02, C07C 2/66, B01J 29/06

(54) **PRODUCTION OF ETHYLTOLUENE**
HERSTELLUNG VON ETHYLTOLUOL
PRODUCTION D'ETHYLTOLUENE

(30) Priority: 05.06.1992 US 894481
(43) Date of publication of application: 22.03.1995
(73) Proprietor: MOBIL OIL CORPORATION, Fairfax, Virginia 22037-0001 (US)
(72) Inventor: CHU, Cynthia, Ting-Wah, Moorestown, NJ 08057 (US); HUANG, Yun, Yang, Voorhees Township, NJ 08043 (US); KLOCKE, Donald, J., Somerdale, NJ 08083 (US)
(74) Representative: Colmer, Stephen Gary
(86) International application number: US9305319
(87) International publication number: WO9325502

(56) References cited:
- US-A- 4 086 287
- US-A- 4 143 084
- US-A- 4 489 214

## Description

This invention relates to a process for the production of ethyltoluene and more particularly, to a process for the production of a mixture of ethyltoluene isomers having a high content of the meta isomer and a very low quantity of the ortho isomer.

Vinyl toluene is an important industrial commodity chemical used for the production of polymers. For certain purposes, it is desirable to produce vinyl toluene with a relatively high content of the meta isomer (m-methyl styrene), typically from 50 to 70% of the total vinyl toluene content. At the same time, a very low content of the ortho isomer (o-methyl styrene) is desired since this material acts as a chain stopper in polymerization. Normally, a maximum content of the ortho isomer of 1.5% and preferably below 1 or 0.5% must be adhered to for a commercially acceptable material.

Vinyl toluene is produced from ethyltoluene by dehydrogenation with the isomer ratio of the vinyl toluene product being directly dependent upon the isomer ratio of the ethyltoluene precursor. Thus, the ethyltoluene should typically contain from 50 to 70% of the meta isomer and not more than 1% ortho isomer with the balance being made up of the para isomer.

Ethyltoluene may be produced by the ethylation of toluene in the presence of an acidic catalyst and for this purpose, Lewis acid type catalysts such as aluminum chloride as well as heterogeneous zeolite catalyst have been used. Homogenous phase Lewis acid catalysts such as aluminum trichloride are effective for the ethylation of toluene under relatively mild conditions to produce an ethyltoluene product in which the isomer ratio is relatively close to the equilibrium ratio of 31.5% para, 50.2% meta and 18.3% ortho. U. S. Patent No. 4,143,084 (Kaeding), for example, discloses (Example 13) a process in which toluene is ethylated in the presence of aluminum chloride to produce ethyltoluene with a p-:m-:o- isomer ratio of 27:60:13. While this ethyltoluene product has the requisite content of the meta isomer, the ortho isomer is present in excessive amounts.

An intermediate pore size zeolite such as ZSM-5 may be used to catalyze the ethylation of toluene, typically using temperatures in the range of 260° to 455°C (500° to 850°F). The isomer ratio of the product varies according to the specific shape selectivity characteristics of the zeolite component of the catalyst. If ZSM-5 is used in its hydrogen form without modification, the ethyltoluene product has an isomer ratio which is relatively close to equilibrium. An example of this is given in US 4086287 where Example 3 shows that a meta content of about 60% may be obtained but that the ortho isomer is present in excessive amounts.

It is possible to modify zeolite catalysts so that selectivity for the para isomer is increased significantly about the equilibrium level. For example, by increasing the crystallite size of ZSM-5 the ratio of para to meta isomer may be raised significantly with a corresponding loss in the amount of the ortho isomer. US 4086287 (Example 4) shows that ortho contents as low as about 1.6% may be obtained with meta contents of about 65% using a large crystal ZSM-5 catalyst. Even here however the content of the ortho isomer is excessive. High temperatures and relatively low space velocities in a commercial process are desirable because they enable more product to be produced in units of given size and from Example 4 there is no indication that higher space velocities will achieve the preferred isomer ratio.

The alpha value of the catalyst in US 4086287 is preferably less than 20, and this may be obtained by steaming.

By suitable modification of the diffusion characteristics of the zeolite, the content of the ortho isomer may be reduced to suitably low levels but this is accompanied by a major increase in the selectivity to the para isomer to the extent that the process is highly regioselective. When ZSM-5 is modified with magnesium, phosphorous or boron, the content of the ortho isomer may be reduced almost to zero but in this case, selectivity for the para isomer is exceptionally high so that the ethyltoluene product does not have the desired balance of the para and meta isomer components. Processes such as this are described in U. S. Patents No. 4,086,287 and 4,143,084.

U.S. Patent No. 4,489,214 (Butler) discloses a process for producing ethyltoluene using a catalytic material described as "silicalite", a material known to be a highly siliceous from of zeolite ZSM-5. See Fyfe et al., "Resolving crystallographically distinct sites in silicalite and ZSM-5 by soid state NMR", Nature 296, 530 (1982) and Olson, D. H. et al., J. Catalysis 61, 390-396 (1980).

In summary, therefore, the problem remains of producing ethyltoluene with a relatively high content of the meta isomer and a low content of the ortho isomer not exceeding 1.5% and preferably less than 0.5%.

We have now devised a process for the production of ethyltoluene which has a relatively high content of the meta isomer and a low content of the ortho isomer. The m-:p- ratio of the product is at least 1:1 (m-:p-) and preferably at least 1.5:1 by weight; the level of the ortho isomer does not, in any event, exceed 1.5% and is usually below 1%. This result is achieved by suitable selection of catalyst and process constraints in the reaction.

The ethyltoluene product is produced by the ethylation of toluene in the presence of an intermediate pore size aluminosilicate zeolite catalyst of controlled acidity produced by steaming. The reaction is carried out at temperatures from 370° to 480°C (700° to 900 °F) with toluene:ethylene ratios from 2:1 to 20:1 and space velocities based on the toluene feed, of 10 to 40 (WHSV). The temperature will normally not exceed 455°C (850°F).

In the present process toluene is reacted with ethylene in the presence of an intermediate pore size steamed aluminosilicate zeolite catalyst. The objective is to produce an ethyltoluene product which has a high content of the meta isomer and a very low content of the ortho isomer. The meta: para ratio of the product is at least 1:1 by weight (equivalent to at least 50 weight percent of the meta-isomer on an ortho-free basis) and is preferably at least 1.2:1 (at least 55 weight percent meta- on an ortho-free basis). In all cases, however, the maximum content of the ortho isomer is to be maintained at 1.5% and should preferably be below 1% or, better, 0.5% by weight if the ethyltoluene product is to be used for a polymerization grade vinyltoluene.

When toluene is reacted with ethylene in the presence of the shape selective heterogeneous catalyst such as ZSM-5, the initial product is probably the paraethyltoluene which is progressively converted to the meta- and then to the ortho- isomers as contact with the acidic catalyst continues. As reaction severity increases, movement towards equilibrium is accelerated correspondingly and it therefore becomes difficult to produce a product containing relatively large amount of the meta isomer while, at the same time maintaining the content of the ortho isomer at very low levels. Normally, as shown by the Kaeding patents mentioned above, production of the meta isomer is accompanied by increasing amount of the ortho isomer as the composition of the reaction product moves toward equilibrium in the presence of the catalyst unless the shape selectivity of the catalyst is modified to the extent that it becomes highly regioselective for the para-isomer, in which case insufficient quantities of the meta isomer are produced.

In the present process, however, the activity and selectivity of the catalyst are modified together and used in combination with selected reaction parameter to produce an ethyltoluene product with the required isomer distribution. In addition to giving the desired high meta content material with a very low ortho content, the proportion of ethylbenzene by-product, produced by disproportionation of the toluene feed, is minimized by controlling the activity of the catalyst to inhibit the disproportionation reaction while maintaining a sufficient contact time (reciprocal space velocity) to shift the composition of the desired ethyltoluene product to the desired isomer distribution.

The catalyst includes aluminosilicate zeolite ZSM-5 as the essential catalytic component. This zeolite, an intermediate pore size zeolite, is matrixed with a binder such as alumina in order to give sufficient strength to the catalyst and in order to avoid binder effects a low acidity material is preferred. The ZSM-5 should have a crystallite size of at least one micron in order to provide the desired diffusion characteristics and crystallite sizes of about 1.5 microns or more e.g. 2 micron are preferred. ZSM-5 with this crystal size may be readily made by conventional techniques using substituted ammonium directing agents such as tetrapropylammonium cations in combination with trimethylammonium cations, as described in U.S. 4,375,458 (Dwyer) to which reference is made for a description of such a technique.

Activity of the zeolite is maintained at a controlled, relatively low level, by steaming the original zeolite in order to reduce the incidence of side reactions such as the toluene disproportionation reaction. The acid activity of the catalyst should be maintained at an alpha value of not more than 80, preferably not greater than 50, more preferably not greater than 40. It has been found that an alpha value of about 30 gives good results.

The alpha value is an approximate indication of the catalytic cracking activity of the catalyst compared to a standard catalyst. The alpha test gives the relative rate constant (rate of normal hexane conversion per volume of catalyst per unit time) of the test catalyst relative to the standard catalyst which is taken as an alpha of 1 (Rate Constant = 0.016 sec ⁻¹). The alpha test is described in U.S. Patent 3,354,078 and in J. Catalysis, 4, 527 (1965); 6, 278 (1966); and 61, 395 (1980), to which reference is made for a description of the test. The experimental conditions of the test used to determine the alpha values referred to in this specification include a constant temperature of 538°C and a variable flow rate as described in detail in J. Catalysis, 61, 395 (1980).

Production of the desired acidic activity level by steaming has been found to be an essential feature of the process: the use of low activity, highly siliceous zeolites does not result in the production of the desired high meta content ethyltoluene product. Normally, a silica:alumina ratio of about 70:1 in the zeolite prior to steaming to the final alpha value will give good results for the finished catalyst.

The reaction is carried out by passing toluene and ethylene together over the ZSM-5 catalyst at temperatures in the range of about 260° to 455°C (about 500° to about 850°F). The contact time (reciprocal space velocity) is maintained at a value which enables the product composition to shift sufficiently to give the desired content of the meta isomer while maintaining the ortho content at the necessary low level. Because reaction temperature and contact time are both related to total reaction severity, a trade off may be affected between temperature and space velocity with decreasing space velocity increasing the content of the meta and ortho isomers. Normally, temperatures will be from about 400° to 455°C about 750° to 850°F with space velocities, based on the toluene feed, from 10 to 40 WHSV.

The relatively high velocities are important. In a commercial process, high space velocities enable more product to be produced in a unit of a given size. Additionally, we discovered that the space velocity contributes to a product mixture that contains high amounts of the meta-isomer and only minimal amounts of the ortho-isomer.

The reaction is not critical with respect to pressure and for reasons of economy it will be convenient to operate at low to moderate pressures, typically up to about 3550 kPa abs about 500 psig although higher pressures may be used if desired. A relatively high ratio of toluene to ethylene is preferred in order to ensure a high selectivity of ethylene to ethyltoluene. Typically, the ratio of toluene to ethylene will be from 2:1 to about 30:1 and in most cases ratios from 10:1 to 20:1 (molar) will be satisfactory.

### Example 1

Three different ZSM-5/alumina catalysts were tested in the ethylation of toluene. The catalysts were as follows:
- Catalyst A:: unsteamed ZSM-5, crystalline size >1 micron, 358 alpha
- Catalyst B:: steamed ZSM-5, crystalline size >1 micron, 70 alpha
- Catalyst C:: steamed ZSM-5, crystalline size >1 micron, 30 alpha.

Catalyst A is not in accordance with the invention. It provides a comparative example. Toluene and ethylene were passed over the catalysts at temperatures from 432° to 446°C (810° to 835°F) at a pressure of 1130 kPa abs (150 psig). The space velocity based on the toluene feed was 130 WHSV. A toluene: ethylene mole ratio of 16 was employed. The results are given in Table 1 below.

**Table 1**

| Ethyltoluene Production over ZSM-5 | | | |
|---|---|---|---|
| Catalyst | A | B | C |
| | 358α | 70α | 30α |
| Temperature, °C (°F)* | 434 (814) | 436 (817) | 444 (832) |
| Pressure, MPa (psig) | 1.13 (150) | 1.13 (150) | 1.13 (150) |
| WHSV (Toluene) | 130 | 130 | 130 |
| Toluene/C2=(mol) | 16 | 16 | 16 |

| Normalized Ethyltoluene % | | | |
|---|---|---|---|
| para | 51.8 | 60.9 | 69.7 |
| meta | 46.7 | 38.7 | 30.0 |
| ortho | 1.5 | 0.4 | 0.3 |

| Liquid Products, wt% | | | |
|---|---|---|---|
| Light Gas | 0.35 | 1.85 | 0.16 |
| Benzene | 4.49 | 0.67 | 0.97 |
| EB | 1.98 | 0.84 | 0.81 |
| Xylenes | 6.82 | 3.37 | 2.58 |
| p-ET | 42.88 | 56.40 | 66.52 |
| m-ET | 38.65 | 35.86 | 28.62 |
| o-ET | 1.21 | 0.34 | 0.32 |
| C10+ Aromatics | 3.62 | 0.67 | - - |
| | | | |
| Total ET | 82.74 | 92.60 | 95.46 |
| Bz + EB + Xy | 13.29 | 4.88 | 4.36 |
| Other | 3.97 | 2.52 | 0.18 |
| [Bz+EB]/Xyl, mol | 1.20 | 0.53 | 0.82 |

| | | | |
|---|---|---|---|
| * Reactor inlet | | | |

### Example 2

The effect of reaction severity (contact time, temperature) was investigated using the steamed ZSM-5 30 alpha catalyst (Catalyst C) above. Toluene ethylation was carried out at temperatures from 404 to 421 °C (760° to about 790°F) (reactor inlet). Space velocity based on the toluene feed varied from 20 to 130 (WHSV). A toluene:ethylene mole ratio of 7 or 16 was used at a pressure of 1.13 mPa (150 psig). No hydrogen was added. The results are shown in Table 2 below.

**Table 2**

| Ethyltoluene Production - Reaction Severity | | | | |
|---|---|---|---|---|
| TOS, hr | 26 | 2 | 6 | 9 |
| Temp. °C (°F)* | 416 (781) | 420 (788) | 419 (787) | 406 (762) |
| WHSV (toluene) | 20 | 40 | 60 | 130 |
| Tol/C₂=(mole) | 7 | 7 | 7 | 16 |
| P, MPa (psig) | 1.13 (150) | 1.13 (150) | 1.13 (150) | 1.13 (150) |

| Normalized Ethyltoluene. % | | | | |
|---|---|---|---|---|
| p-ET | 33.39 | 55.04 | 66.31 | 77.43 |
| m-ET | 65.60 | 44.61 | 33.42 | 22.39 |
| o-ET | 1.01 | 0.35 | 0.27 | 0.18 |

| Liquid Products, wt% | | | | |
|---|---|---|---|---|
| Light gas | 0.30 | 0.14 | - | 0.17 |
| Benzene | 1.98 | 0.56 | 0.41 | 0.50 |
| EB | 1.14 | 0.79 | 0.67 | 0.83 |
| xyl | 3.27 | 1.45 | 1.44 | 2.00 |
| p-ET | 29.96 | 52.29 | 64.51 | 74.72 |
| m-ET | 58.87 | 42.39 | 32.51 | 21.61 |
| o-ET | 0.91 | 0.33 | 0.26 | 0.17 |
| C₁₀+ Aromatics | 3.57 | 2.05 | 0.21 | - |
| | | | | |
| Total ET | 89.74 | 95.01 | 97.33 | 96.50 |
| Bz+EB+Xyl | 6.39 | 2.80 | 2.52 | 3.33 |
| Other | 3.87 | 2.19 | 0.15 | 0.17 |
| [Bz+EB]/Xyl, mol | 1.17 | 1.08 | 0.93 | 0.75 |

| | | | | |
|---|---|---|---|---|
| * Reactor Inlet . | | | | |

The results in Table 3 show that production of the meta isomer increases with increasing contact time (lower numerical space velocity) but is accompanied by an increase in the level of the undesired ortho isomer. By selection of reaction severity the ortho isomer may be maintained below 0.5% while obtaining a high level of the meta isomer.

### Example 3

Toluene was ethylated over the steamed, 30 alpha ZSM-5 catalyst with hydrogen co-feed at total system pressures (reactor inlet) up to 1820 kpa abs (250 psig) the results are shown in Table 3 below.

The results show that the hydrogen co-feed does not affect the distribution of the ethyltoluene isomers significantly (compare Table 2 above), but slightly decreases the disproportionation activity. In these studies, however, relatively long contact times were used and the content of the undesired ortho ethyltoluene isomer may exceed acceptable levels when operating at greater severity.

**Table 3**

| Ethyltoluene Production - H₂ Co-Feed | | | |
|---|---|---|---|
| TOS, hr | 26 | 7 | 10 |
| Temp. °C (°F)* | 416 (781) | 436 (816) | 432 (810) |
| WHSV (toluene) | 20 | 20 | 20 |
| Tol/C₂=/H₂ (mole) | 7/1 | 7/1 | 7/1/5 |
| P, MPa (psig)* | 1.13 (150) | 1.13 (150) | 1.82 (250) |

| Normalized Ethyltoluene, % | | | |
|---|---|---|---|
| p-ET | 33.39 | 31.63 | 34.53 |
| m-ET | 65.60 | 67.01 | 64.47 |
| o-ET | 1.01 | 1.36 | 1.00 |

| Liquid Products, wt% | | | |
|---|---|---|---|
| Light gas | 0.30 | 0.66 | 0.08 |
| Benzene | 1.98 | 3.37 | 1.96 |
| EB | 1.14 | 1.54 | 1.13 |
| xyl | 3.27 | 4.68 | 3.84 |
| p-ET | 29.96 | 27.23 | 31.23 |
| m-ET | 58.87 | 57.69 | 58.31 |
| o-ET | 0.91 | 1.17 | 0.90 |
| C₁₀+ Aromatics | 3.57 | 3.66 | 2.63 |
| | | | |
| Total ET | 89.74 | 86.09 | 90.44 |
| Bz+EB+Xyl | 6.39 | 9.59 | 6.93 |
| Other | 3.87 | 4.32 | 2.63 |
| [Bz+EB]/Xyl, mol | 1.17 | 1.31 | 0.99 |

| | | | |
|---|---|---|---|
| * Reactor inlet | | | |

### Example 4

Three runs were made using a low alpha (alpha = 16) highly siliceous ZSM-5 with a crystal size of 2 microns. The reaction was carried out at higher temperatures in order to compensate for the lower acidity of the catalyst, with the temperature varying from 438 to 455° C (820° to 850°F) at the reactor inlet. The reaction was carried out in the absence of hydrogen at a toluene:ethylene molar ratio of 16:1. Space velocities varied inversely with temperature from 60 to 130. The results are shown in Table 4 below.

**Table 4**

| Ethyltoluene Production - High Silica ZSM-5 | | | |
|---|---|---|---|
| TOS, hr | 3 | 6 | 9 |
| Temp. °C (°F)* | 439 (823) | 452 (845) | 454 (850) |
| WHSV (toluene) | 130 | 80 | 60 |
| Tol/C₂= (mole) | 16 | 16 | 16 |

| Normalized Ethyltoluene, % | | | |
|---|---|---|---|
| p-ET | 79.57 | 72.86 | 67.36 |
| m-ET | 20.05 | 26.80 | 32.01 |
| o-ET | 0.38 | 0.34 | 0.63 |

| Liquid Products, wt% | | | |
|---|---|---|---|
| Light gas | 0.70 | 0.60 | 0.72 |
| Benzene | 1.29 | 2.18 | 3.64 |
| EB | 1.05 | 1.41 | 1.64 |
| Xyl | 3.85 | 5.01 | 6.38 |
| p-ET | 74.10 | 66.16 | 59.02 |
| m-ET | 18.67 | 24.34 | 28.05 |
| o-ET | 0.35 | 0.31 | 0.55 |
| C₁₀+ Aromatics | | | |
| | | | |
| Total ET | 93.12 | 90.81 | 87.07 |
| Bz+EB+Xyl | 6.19 | 8.60 | 11.66 |
| Other | 0.69 | 0.59 | 0.72 |
| [Bz+EB]/Xyl, mol | 0.72 | 1.00 | 1.05 |

| | | | |
|---|---|---|---|
| * Reactor inlet | | | |

The results in Table 4 above show that a low content of the ortho isomer may be obtained in the product using the highly siliceous zeolite but that this is not accompanied by an adequately high level for the meta isomer.

### Example 5

The effect of the ZSM-5 crystal size was investigated with two ZSM-5 catalysts. The first catalyst (Catalyst D) had an alpha value of 50 and a zeolite crystal size of less than 0.05 µm and the second (Catalyst E) an alpha value of 18 and a crystal size between 0.1 and 1 µm. The two catalysts were used to ethylate toluene at temperatures from about 415 to 460°C (about 780° to 860°F) in the absence of hydrogen at a pressure of 1135 kPaa (150 psig). The results for Catalyst D are given in Table 5 and for Catalyst E in Table 6 below.

**Table 5**

| Ethyltoluene Synthesis - 50 alpha ZSM-5 | |
|---|---|
| TOS, hr | 8 |
| Temp, °C (°F)* | 436 (816) |
| WHSV (toluene) | 130 |
| Tol/C₂= | 7/1 |
| P, MPa (psig) | 1.13 (150) |

| Normalized Ethyltoluene, % | |
|---|---|
| p-ET | 31.31 |
| m-ET | 63.26 |
| o-ET | 5.44 |

| Liquid Products, wt% | |
|---|---|
| Light gas | 0.46 |
| Benzene | 2.19 |
| EB | 4.47 |
| Xyl | 6.58 |
| p-ET | 27.03 |
| m-ET | 54.58 |
| o-ET | 4.70 |
| | |
| Total ET | 86.31 |
| Bz+EB+Xyl | 13.24 |
| Other | 0.45 |
| [Bz+EB]/Xyl, mole | 1.13 |

**Table 6**

| Ethyltoluene Synthesis - 18 alpha ZSM-5 | | |
|---|---|---|
| TOS, hr | 2 | 12 |
| Temp. °C (°F)* | 433 (812) | 419 (787) |
| WHSV (Toluene) | 20 | 20 |
| Tol/C₂= /H₂ | 7/1 | 7/1 |
| P, MPa (psig) | 1.13 (150) | 1.13 (150) |

| Normalized Ethyltoluene, % | | |
|---|---|---|
| p-ET | 28.61 | 30.05 |
| m-ET | 63.62 | 66.21 |
| o-ET | 7.87 | 3.73 |

| Liquid Products, wt% | | |
|---|---|---|
| Light gas | 0.14 | 0.08 |
| Benzene | 1.79 | 1.31 |
| EB | 1.00 | 0.77 |
| Xyl | 2.76 | 2.00 |
| p-ET | 26.36 | 28.38 |
| m-ET | 58.52 | 62.46 |
| o-ET | 7.25 | 3.54 |
| C₁₀+ Aromatics | 2.28 | 1.46 |
| | | |
| Total ET | 92.13 | 94.38 |
| Bz+EB+Xyl | 5.55 | 4.08 |
| Other | 2.32 | 1.54 |
| [Bz+EB]/Xyl, mol | 1.24 | 1.27 |

| | | |
|---|---|---|
| * Reactor inlet | | |

The results given above show that the crystal size of the zeolite component of the catalyst affected the isomer distribution of the ethyltoluene product: the catalysts with the low acidity, smaller crystal ZSM-5 do not achieve the low ortho-isomer content which is attainable with the low acidity, larger crystal catalysts of Example 1 above (Catalysts B, C), under comparable conditions.

### Example 6

The effect of increasing space velocity on the proportion of meta-isomer in the product was investigated using a steamed ZSM-5 catalyst having an alpha value of about 30.

The four cases reported in Table 7 show the results obtained at four different times on stream (OS) in a two-bed operation at temperatures within the range of 413-427°C (775°-800°F). The space velocity of the reactants, reported as WHSV toluene, was varied from about 15 to about 42 to obtain the results shown.

**Table 7**

| Mobil Ethyltoluene (MET) Catalyst - Two-Bed Operation | | | | |
|---|---|---|---|---|
| Case | A | B | C | D |
| Hours OS | 8 | 44 | 46 | 68 |

| T(avg) °C (°F) | | | | |
|---|---|---|---|---|
| Bed #1 | 413 (775) | 414 (778) | 425 (797) | 427 (800) |
| Bed #2 | 404 (760) | 406 (763) | 467 (783) | 424 (796) |
| Tol WHSV | 41.8 | 21.2 | 21.8 | 15.2 |
| Tol/C₂=H₂,mol | 8/1/7 | 5/1/7 | 6/1/8 | 7/1/18 |
| Press, MPa (psig) | 1.82 (250) | 1.84 (252) | 1.84 (252) | 1.48 (200) |
| | | | | |

| ET Isomers, % | | | | |
|---|---|---|---|---|
| m | 43.4 | 51.1 | 54.1 | 57.9 |
| p | 56.2 | 48.3 | 45.1 | 41.0 |
| o | 0.5 | 0.6 | 0.8 | 1.1 |
| | | | | |
| Total ET, wt% | 14.1 | 22.3 | 18.1 | 19.5 |

| Byproduct, wt% | | | | |
|---|---|---|---|---|
| Bz+EB+Xy | 0.89 | 1.05 | 1.00 | 1.15 |
| Hvy | 0.17 | 0.44 | 0.24 | 0.23 |
| | | | | |
| ET Prod, lb/hr | 9,500 | 7,800 | 6,000 | 4,000 |

The results show that as the space velocity increases, the proportion of the meta-isomer in the product, decreases. At the same time, the proportion of the ortho-isomer decreases. The effect of increasing space velocity is therefore to move the composition of the isomer mixture away from equilibrium values, while a decreased velocity will produce a trend towards equilibrium. The content of the ortho-isomer in the product tends to increase along with the meta at progressively lower space velocities.

## Claims

1. A process for the production of an ethyltoluene isomer mixture of high m-isomer and very low o-isomer content, which comprises ethylating toluene with ethylene in the presence of a catalyst comprising ZSM-5 having a crystallite size of at least one micron and which has been steamed to an alpha value not more than 80, at a temperature from 370° to 480°C (700° to 900°F) and a space velocity, based on the toluene feed, from 10 to 40 WHSV, to produce a reaction product comprising a mixture of isomers of ethyltoluene in which the weight ratio of the m-isomer to the p-isomer is at least 1:1, and of the o-isomer is not more than 1.5 wt%.

2. A process according to claim 1 in which the weight ratio of the m-isomer to the p-isomer in the ethyltoluene isomer mixture is at least 1.2:1.

3. A process according to claim 1 in which the content of the o-isomer in the ethyltoluene isomer mixture is not more than 1 wt%.

4. A process according to claim 1 in which the content of the o-isomer in the ethyltoluene isomer mixture is not more than 0.5 wt%.

5. A process according to claim 1 in which the crystallite size of the ZSM-5 is at least 2 microns.

6. A process according to claim 1 in which the catalyst has an alpha value not greater than 50.

7. A process according to claim 5 in which the catalyst has an alpha value not greater than 40.

8. A process according to claim 1 in which the toluene is reacted with the ethylene at a temperature from 399 to 480°C (750° to 850°F).

## Patentansprüche

1. Verfahren zur Herstellung einer Ethyltoluol-Isomermischung mit hohem Gehalt an m-Isomer und sehr geringem Gehalt an o-Isomer, welches umfaßt: Ethylieren von Toluol mit Ethylen in Gegenwart eines Katalysators, der ZSM-5 mit einer Kristallitgröße von mindestens 1 µm umfaßt, und der auf einen α-Wert von nicht mehr als 80 dampfbehandelt wurde, bei einer Temperatur von 370 bis 480°C (700 bis 900°F) und einer Raumgeschwindigkeit auf der Basis der Toluolbeschickung von 10 bis 40 WHSV, wodurch ein Reaktionsprodukt hergestellt wird, das eine Mischung der Ethyltoluol-Isomere umfaßt, bei der das Gewichtsverhältnis des m-Isomers zum p-Isomer mindestens 1:1 und des o-Isomers nicht mehr als 1,5 Gew.-% beträgt.

2. Verfahren nach Anspruch 1, wobei das Gewichtsverhältnis des m-Isomers zum p-Isomer in der Ethyltoluol-Isomermischung mindestens 1,2:1 beträgt.

3. Verfahren nach Anspruch 1, wobei der Gehalt des o-Isomers in der Ethyltoluol-Isomermischung nicht mehr als 1 Gew.-% beträgt.

4. Verfahren nach Anspruch 1, wobei der Gehalt des o-Isomers in der Ethyltoluol-Isomermischung nicht mehr als 0,5 Gew.-% beträgt.

5. Verfahren nach Anspruch 1, wobei die Kristallitgröße von ZSM-5 mindestens 2 µm beträgt.

6. Verfahren nach Anspruch 1, wobei der Katalysator einen α-Wert von nicht mehr als 50 aufweist.

7. Verfahren nach Anspruch 5, wobei der Katalysator einen α-Wert von nicht mehr als 40 aufweist.

8. Verfahren nach Anspruch 1, wobei Toluol bei einer Temperatur von 399 bis 480°C (750 bis 850°F) mit Ethylen umgesetzt wird.

## Revendications

1. Un procédé de fabrication d'un mélange d'isomèreS d'éthyltoluène à haute teneur en isomère meta- et à très faible teneur en isomère ortho-, qui comprend l'éthylation du toluène par l'éthylène en présence d'un catalyseur comprenant une ZSM-5 présentant une dimension de cristallite d'au moins un micron et qui a été traitée à la vapeur d'eau pour lui conférer une valeur de alpha ne dépassant 80, à une température de 370 à 480°C (700 à 900°F) et à une vitesse spatiale, exprimée par rapport à la charge de toluène, comprise entre 10 et 40 WHSV, pour obtenir un produit réactionnel comprenant un mélange d'isomères de l'éthyltoluène dans lequel le rapport pondéral de l'isomère méta- à l'isomère para- est d'au moins 1/1 et de moins de 1,5 % en poids d'isomère ortho-.

2. Un procédé selon la revendication 1, dans lequel le rapport pondéral de l'isomère méta- à l'isomère para- dans le mélange d'isomères d'éthyltoluène est d'au moins 1,2/1.

3. Un procédé selon la revendication 1, dans lequel la teneur en isomère ortho- du mélange d'isomères d'éthyltoluène n'est pas supérieure à 1 % en poids.

4. Un procédé selon la revendication 1, dans lequel la teneur en isomère ortho- dans le mélange d'isomères d'éthyltoluène n'est pas supérieure à 0,5 % en poids.

5. Un procédé selon la revendication 1, dans lequel la dimension des cristallites de la ZSM-5 est d'au moins 2 microns.

6. Un procédé selon la revendication 1, dans lequel le catalyseur a une valeur de alpha non supérieure à 50.

7. Un procédé selon la revendication 5, dans lequel le catalyseur présente une valeur de alpha non supérieure à 40.

8. Un procédé selon la revendication 1, dans lequel on fait réagir le toluène avec l'éthylène à une température comprise entre 399 et 480°C (750 à 850°F).
